# EUROPÄISCHE PATENTSCHRIFT

(11) **EP 1 585 747 B1**
(45) Veröffentlichungstag und Bekanntmachung des Hinweises auf die Patenterteilung: **13.09.2006**
(21) Anmeldenummer: 03775290.4
(22) Anmeldetag: 04.11.2003
(51) Int. Cl.: C07D 487/04

(54) **5-ALKYL-7-AMINOTRIAZOLOPYRIMIDINE,VERFAHREN UND ZWISCHENPRODUKTE ZU IHRER HERSTELLUNG ,SIE ENTHALTENDE MITTEL SOWIE IHRE VERWENDUNG ZUR BEKÄMPFUNG VON SCHADPILZEN**
5-ALKYL-7-AMINOTRIAZOLOPYRIMIDINES, METHODS AND INTERMEDIATES SUITABLE FOR THEIR PRODUCTION, AGENTS CONTAINING THEM AND THEIR USE FOR CONTROLLING HARMFUL FUNGI
5-ALKYL-7-AMINOTRIAZOLOPYRIMIDINES, PROCEDES ET PRODUITS INTERMEDIAIRES POUR LEUR PRODUCTION, AGENTS LES CONTENANT ET LEUR UTILISATION POUR LUTTER CONTRE DES CHAMPIGNONS NOCIFS

(30) Priorität: 07.11.2002 DE 10252261
(43) Veröffentlichungstag der Anmeldung: 19.10.2005
(73) Patentinhaber: BASF Aktiengesellschaft, 67056 Ludwigshafen (DE)
(72) Erfinder: TORMO I BLASCO, Jordi, 69469 Weinheim (DE); BLETTNER, Carsten, 68165 Mannheim (DE); MÜLLER, Bernd, 67227 Frankenthal (DE); GEWEHR, Markus, 56288 Kastellaun (DE); GRAMMENOS, Wassilios, 67071 Ludwigshafen (DE); GROTE, Thomas, 67157 Wachenheim (DE); GYPSER, Andreas, 68159 Mannheim (DE); RHEINHEIMER, Joachim, 67063 Ludwigshafen (DE); SCHÄFER, Peter, 67308 Ottersheim (DE); SCHIEWECK, Frank, 67258 Hessheim (DE); SCHWÖGLER, Anja, 68165 Mannheim (DE); AMMERMANN, Eberhard, 64646 Heppenheim (DE); STRATHMANN, Siegfried, 67117 Limburgerhof (DE); SCHÖFL, Ulrich, 68782 Brühl (DE); STIERL, Reinhard, 67251 Freinsheim (DE)
(86) Internationale Anmeldenummer: PCT/EP2003/012277
(87) Internationale Veröffentlichungsnummer: WO 2004/041825

(56) Entgegenhaltungen:
- EP-A- 0 141 317
- WO-A-02/083677

## Beschreibung

Die vorliegende Erfindung betrifft 5-Alkyl-7-aminotriazolopyrimidine der Formel I, 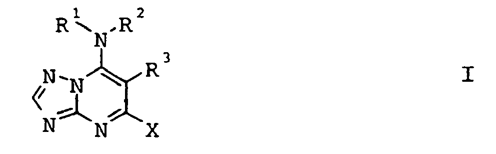 in der die Substituenten die folgenden Bedeutungen haben:
- R¹,R²: Wasserstoff, C₁-C₁₀-Alkyl, C₂-C₁₀-Alkenyl, C₂-C₁₀-Alkinyl, C₁-C₁₀-Haloalkyl, C₃-C₈-Cycloalkyl, Phenyl, Naphthyl; oder
5- oder 6-gliedriges gesättigtes, ungesättigtes oder aromatisches Heterocyclyl, enthaltend ein bis vier Stickstoffatome oder ein bis drei Stickstoffatome und ein Schwefel-oder Sauerstoffatom; oder
R¹ und R² können zusammen mit dem Stickstoffatom, das sie verbindet, einen 5- oder 6-gliedrigen Ring bilden, der ein bis vier Stickstoffatome oder ein bis drei Stickstoffatome und ein Schwefel- oder Sauerstoffatom enthält;
R¹ und R² können, wenn ungleich Wasserstoff, unabhängig voneinander teilweise oder vollständig halogeniert sein und/oder einen bis drei Reste aus der Gruppe R^{a} tragen
R^{a} Cyano, Nitro, Hydroxy, C₁-C₆-Alkyl, C₁-C₆-Haloalkyl, C₃-C₆-Cycloalkyl, C₁-C₆-Alkoxy, C₁-C₆-Haloalkoxy, C₁-C₆-Alkylthio, C₁-C₆-Alkylamino, Di-C₁-C₆-alkylamino, C₂-C₆-Alkenyl, C₂-C₆-Alkenyloxy, C₂-C₆-Alkinyl, C₃-C₆-Alkinyloxy und gegebenenfalls halogeniertes Oxy-C₁-C₄-alkylenoxy; wobei diese aliphatischen, alicyclischen oder aromatischen Gruppen ihrerseits partiell oder vollständig halogeniert sein oder eine bis drei Gruppen R^{b} tragen können:
R^{b} Halogen, Cyano, Nitro, Hydroxy, Mercapto, Amino, Carboxyl, Aminocarbonyl, Aminothiocarbonyl, Alkyl, Haloalkyl, Alkenyl, Alkenyloxy, Alkinyloxy, Alkoxy, Halogenalkoxy, Alkylthio, Alkylamino, Dialkylamino, Formyl, Alkylcarbonyl, Alkylsulfonyl, Alkylsulfoxyl, Alkoxycarbonyl, Alkylcarbonyloxy, Alkylaminocarbonyl, Dialkylaminocarbonyl, Alkylaminothiocarbonyl, Dialkylaminothiocarbonyl, wobei die Alkylgruppen in diesen Resten 1 bis 6 Kohlenstoffatome enthalten und die genannten Alkenyloder Alkinylgruppen in diesen Resten 2 bis 8 Kohlenstoffatome enthalten; und/oder einen bis drei der folgenden Reste:
Cycloalkyl, Cycloalkoxy, Heterocyclyl, Heterocyclyloxy, wobei die cyclischen Systeme 3 bis 10 Ringglieder enthalten; Aryl, Aryloxy, Arylthio, Aryl-C₁-C₆-alkoxy, Aryl-C₁-C₆-alkyl, Hetaryl, Hetaryloxy, Hetarylthio, wobei die Arylreste vorzugsweise 6 bis 10 Ringglieder, die Hetarylreste 5 oder 6 Ringglieder enthalten, wobei die cyclischen Systeme partiell oder vollständig halogeniert oder durch Alkyl- oder Haloalkylgruppen substituiert sein können;
- R³: C₃-C₁₄-Cycloalkyl oder C₆-C₁₄-Bicycloalkyl, wobei R³ unsubstituiert oder teilweise oder vollständig halogeniert sein kann und/oder ein bis drei Reste aus der Gruppe R^{a} tragen kann; und
- X: C₁-C₆-Alkyl oder C₁-C₂-Halogenalkyl;
sowie deren Salze.

Zusätzlich betrifft die Erfindung Verfahren und Zwischenprodukte zur Herstellung der Verbindungen I, sowie Mittel und die Verwendung der Verbindungen I zur Bekämpfung von phytopathogenen Schadpilzen.

6-Aryl-Triazolopyrimidine werden in WO 98/46608 und EP-A 550 113 offenbart. Durch aromatische Gruppen speziell substituierte 6-Benzyl-Triazolopyrimidine mit pharmazeutischer Wirkung sind aus US 5,231,094 und US 5,387,747 bekannt. EP-A 141 317 offenbart 6-Aryl- und 6-Arylalkyl-7-aminotriazolopyrimidine, welche in 5-Position einen Alkylrest tragen können. 6-Cycloalkyltriazolopyrimidine mit diversen Resten in 5-Position werden in EP-A 613 900 genannt. 5-Alkyl-6-phenyl-7-aminotriazolopyrimidine sind aus US 5,994,360 bekannt.

Die in WO 98/46608, EP-A 550 113, EP-A 141 317, EP-A 613 900 und US 5,994,360 beschriebenen Verbindungen sind als Pflanzenschutzmittel gegen Schadpilze geeignet.

Ihre Wirkung ist jedoch in vielen Fällen nicht zufriedenstellend. Daher lag als Aufgabe zugrunde, Verbindungen mit verbesserter Wirksamkeit zu finden.

Demgemäß wurden die 5-Alkyl-7-aminotriazolopyrimidine der Formel I gefunden. Weiterhin wurden Zwischenprodukte und Verfahren zur Herstellung der Verbindungen I, sowie die Verwendung der Verbindungen I und diese enthaltende Mittel zur Bekämpfung von phytopathogenen Schadpilzen gefunden.

Die Verbindungen der Formel I unterscheiden sich von den aus den oben genannten Schriften bekannten Verbindungen durch die Kombination der 5-Alkylgruppe mit der als mono- oder bicyclisches Cycloalkyl ausgestalteten Gruppe R³ am Triazolopyrimidingerüst.

7-Aminotriazolopyrimidine der Formel I, sind vorteilhaft erhältlich durch Umsetzung von 3-Amino-1,2,4-triazol mit Dicarbonylverbindungen der Formel II, wobei A für C₁-C₁₀-Alkoxy, insbesondere für C₁-C₄-Alkyl steht und R³ und X wie für Formel I definiert sind, zu 7-Hydroxytriazolopyrimidinen der Formel III: 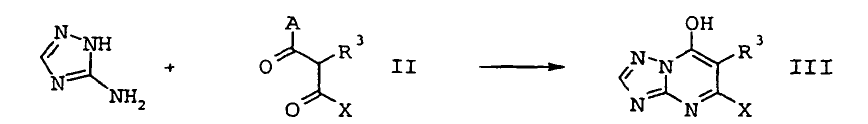 Diese Umsetzung erfolgt üblicherweise bei Temperaturen von 25°C bis 210°C, vorzugsweise 120°C bis 180°C, in Gegenwart einer Base [vgl. EP-A-770 615].

Als Basen kommen allgemein organische Basen, z.B. tertiäre Amine wie Trimethylamin, Triethylamin, Triisopropylethylamin, Tributylamin und N-Methylpiperidin, Pyridin in Betracht. Besonders bevorzugt werden Triethylamin und Tributylamin.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, II in einem Überschuß bezogen auf das Aminotriazol einzusetzen.

Die für die Herstellung der Verbindungen I benötigten Ausgangsstoffe sind in der Literatur bekannt oder können gemäß der zitierten Literatur hergestellt werden [Heterocycl. 1996, S.1031; Tetrahedron Lett. 1966, Bd.24, S.2661; Chem. Pharm. Bull. 1961, S.801] oder sind kommerziell zugänglich.

Anschließend setzt man die 7-Hydroxytriazolopyrimidine der Formel III mit einem Halogenierungsmittel zu 7-Halogentriazolopyrimidinen der Formel IV um: 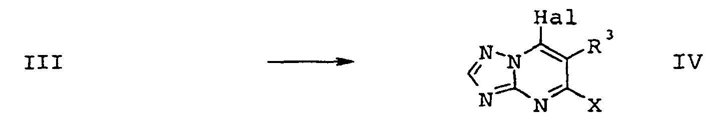 Diese Umsetzung erfolgt üblicherweise bei Temperaturen von 0°C bis 150°C, vorzugsweise 80°C bis 125°C, in einem inerten organischen Lösungsmittel oder ohne Lösungsmittel [vgl. EP-A-770 615].

Als Halogenierungsmittel kommen bevorzugt Bromierungs- oder Chlorierungsmittel, wie beispielsweise Phosphoroxybromid oder Phosphoroxychlorid, in Substanz oder in Anwesendheit eines Lösungsmittels in Frage.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, besonders bevorzugt Toluol, o-, m- und p-Xylol. Es können auch Gemische der genannten Lösungsmittel verwendet werden.

Die Halogentriazolopyrimidine der Formel IV werden mit einem Amin der Formel V zu 7-Aminotriazolopyrimidinen der Formel I umgesetzt. 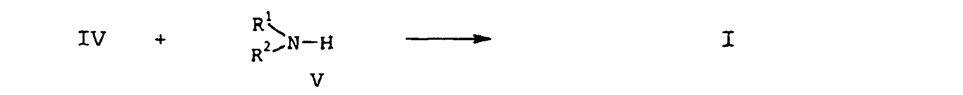 Diese Umsetzung erfolgt üblicherweise bei Temperaturen von 0°C bis 70°C, vorzugsweise 10°C bis 35°C, in einem inerten organischen Lösungsmittel in Gegenwart einer Base [vgl.EP-A 550 113].

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran.

Als Basen kommen allgemein anorganische Verbindungen wie Alkalimetall- und Erdalkalimetallhydroxide, Alkalimetall- und Erdalkalimetalloxide, Alkalimetall- und Erdalkalimetallhydride, Alkalimetallamide, Alkalimetall- und Erdalkalimetallcarbonate wie Lithiumcarbonat, Kaliumcarbonat und Calziumcarbonat sowie Alkalimetallhydrogencarbonate wie Natriumhydrogencarbonat, metallorganische Verbindungen, insbesondere Alkalimetallalkyle, Alkylmagnesiumhalogenide sowie Alkalimetall- und Erdalkalimetallalkoholate und Dimethoxymagnesium, außerdem organische Basen, z.B. tertiäre Amine wie Trimethylamin, Triethylamin, Tri-isopropylethylamin und N-Methylpiperidin, Pyridin, substituierte Pyridine sowie bicyclische Amine in Betracht. Besonders bevorzugt werden Triethylamin, Kaliumcarbonat und Natriumcarbonat.

Die Basen werden im allgemeinen in katalytischen Mengen eingesetzt, sie können aber auch äquimolar, im Überschuß oder gegebenenfalls als Lösungsmittel verwendet werden. Alternativ dazu kann ein Überschuß der Verbindung V als Base dienen.

Die Edukte werden im allgemeinen in äquimolaren Mengen miteinander umgesetzt. Es kann für die Ausbeute vorteilhaft sein, V in einem Überschuß bezogen auf IV einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen-und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Sofern einzelne Verbindungen I nicht auf den voranstehend beschriebenen Wegen zugänglich sind, können sie durch Derivatisierung anderer Verbindungen I hergestellt werden.

7-Hydroxy- und 7-Halogentriazolopyrimidine der Formeln III und IV, wobei X und R³ die Bedeutung wie in Formel I hat und Hal Halogen, insbesondere Chlor oder Brom bedeutet, sind neu.

Bei den in den vorstehenden Formeln angegebenen Definitionen der Symbole wurden Sammelbegriffe verwendet, die allgemein repräsentativ für die folgenden Substituenten stehen:
**Halogen**: Fluor, Chlor, Brom und Jod;
**Alkyl**: gesättigte, geradkettige oder verzweigte Kohlenwasserstoffreste mit 1 bis 4, 6, 8 oder 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Haloalkyl**: geradkettige oder verzweigte Alkylgruppen mit 1 bis 10 Kohlenstoffatomen (wie vorstehend genannt), wobei in diesen Gruppen teilweise, beispielsweise ein- bis dreifach, oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkenyl**: ungesättigte, geradkettige oder verzweigte Kohlenwasserstoffreste mit 2 bis 6 oder 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-lpropenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1propenyl und 1-Ethyl-2-methyl-2-propenyl;
**Alkinyl:** geradkettige oder verzweigte Kohlenwasserstoffgruppen mit 2 bis 6 oder 10 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Cycloalkyl:** monocyclische, gesättigte Kohlenwasserstoffgruppen mit 3 bis 5, 6,oder 8 Kohlenstoffringgliedern, z.B. C₃-C₈-Cycloalkyl wie Cyclopropyl, Cyclobutyl, Cyclopentyl, Cyclohexyl, Cycloheptyl und Cyclooctyl;
**Bicycloalkyl**: bicyclische, gesättigte Kohlenwasserstoffgruppen mit 6 bis 14, insbesondere 7 oder 10 Kohlenstoffringgliedern, bestehend aus annelierten 5-, 6- und/oder 7-gliederigen Ringsystemen.
**5- oder 6-gliedriges Heterocyclyl (gesättigtes Heterocyclyl),** enthaltend ein bis drei Stickstoffatome und/oder ein Sauerstoff-oder Schwefelatom oder ein oder zwei Sauerstoff- und/oder Schwefelatome, z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 2-Pyrrolin-2-yl, 2-Pyrrolin-3-yl, 3-Pyrrolin-2-yl, 3-Pyrrolin-3-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl und 2-Piperazinyl;
**5- oder 6-gliedriges Heterocyclyl (ungesättigtes Heterocyclyl),** enthaltend ein bis drei Stickstoffatome und/oder ein Sauerstoff-oder Schwefelatom oder ein oder zwei Sauerstoff- und/oder Schwefelatome und eine oder zwei C=C-Doppelbindungen, z.B. 3,6-Dihydro-2H-pyridin-1-yl oder 2,5-Dihydropyrrol-1-yl;
**5-gliedriges Heteroaryl (aromatisches Heterocyclyl)**, enthaltend ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, und 1,3,4-Triazol-2-yl;
**6-gliedriges Heteroaryl (aromatisches Heterocyclyl),** enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl und 2-Pyrazinyl;
**Oxyalkylenoxy:** divalente unverzweigte Ketten aus 1 bis 3 CH₂-Gruppen, wobei beide Valenzen über ein Sauerstoffatom an das Gerüst gebunden ist, z.B. OCH₂O, OCH₂CH₂O und OCH₂CH₂CH₂O;

Die Verbindungen der Formel I können auch in Form ihrer landwirtschaftlich brauchbaren Salze vorliegen, wobei es in der Regel nicht auf die Art des Salzes ankommt. Im Allgemeinen kommen die Salze derjenigen Kationen oder die Säureadditionssalze derjenigen Säuren in Betracht, deren Kationen beziehungsweise Anionen die fungizide Wirkung der Verbindungen I nicht negativ beeinträchtigen.

Es kommen als Kationen insbesondere Ionen der Alkalimetalle, vorzugsweise Lithium, Natrium und Kalium, der Erdalkalimetalle, vorzugsweise.Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di(2-hydroxyeth-1-yl)ammonium, Trimethylbenzylammonium, des weiteren, Phosphoniumionen, Sufoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionssalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexaflourosilikat, Hexafluorophosphat, Benzoat sowie die Anionen von C₁-C₄-Alkansäuren, vorzugsweise Formiat, Acetat, Propionat und Butyrat.

Sofern R¹ und/oder R² ein Chiralitätszentrum aufweist, sind die (R)- und (S)-Isomere und die Razemate der Verbindungen der Formel I in den Umfang der Erfindung eingeschlossen.

Die besonders bevorzugten Ausführungsformen der Zwischenprodukte in Bezug auf die Variablen entsprechen denen der Reste R³ und X der Formel I.

Im Hinblick auf ihre bestimmungsgemäße Verwendung der 5-Alkyl-7-aminotriazolopyrimidine der Formel I sind die folgenden Bedeutungen der Substituenten, und zwar jeweils für sich allein oder in Kombination, besonders bevorzugt:

Verbindungen I sind bevorzugt, in denen R¹ und R² für Wasserstoff, C₁-C₁₀-Alkyl oder C₁-C₆-Haloalkyl, insbesondere für Wasserstoff, C₁-C₆-Alkyl, C₁-C₄-Haloalkyl, besonders bevorzugt für Wasserstoff, 1-Methylpropyl, Isopropyl oder 1,1,1-Trifluor-2-propyl steht.

Bevorzugt sind auch Verbindungen I, in denen R¹ und R² zusammen mit dem Stickstoffatom, das sie verbindet, einen 5-oder 6-gliedrigen Ring bilden, der ein Sauerstoff- oder Schwefelatom enthalten kann, wie Pyrrolidin-1-yl, Pyrrol-1-yl, Pyrazol-1-yl, Imidazol-1-yl, Piperidin-1-yl, Morpholin-4-yl, Thiomorpholin-4-yl, 3,6-Dihydro-2H-pyridin-1-yl, 2,5-Dihydropyrrol-1-yl, wobei die genannten Reste durch eine bis drei Gruppen R^{a}, insbesondere durch C₁-C₄-Alkyl, wie beispielsweise Methyl oder Ethyl, substituiert sein können. Besonders bevorzugt sind Verbindungen I, in denen R¹ und R² gemeinsam eine 4-Methylpiperidin-1-yl-Gruppe bilden.

Insbesondere bevorzugt werden Verbindungen I, in denen R¹ nicht Wasserstoff bedeutet.

Daneben werden auch Verbindungen I besonders bevorzugt, in denen R¹ Wasserstoff, C₁-C₆-Alkyl oder C₁-C₄-Haloalkyl und R² Wasserstoff oder C₁-C₄-Alkyl, insbesondere Wasserstoff, bedeutet.

Weiterhin werden Verbindungen I bevorzugt, in denen R¹ und R² keine Gruppen R^{b} tragen, insbesondere solche, in denen R¹ und R² keine Gruppen R^{a} tragen.

Ein weiterer bevorzugter Gegenstand sind Verbindungen I, in denen R¹ und R² Wasserstoff bedeuten.

Besonders werden auch Verbindungen I bevorzugt, in denen R³ C₃-C₁₂-Cycloalkyl, bevorzugt Cyclopropyl, Cyclopentyl, Cyclohexyl, Cyclooctyl oder Cyclododecyl oder Bicycloheptyl bedeuten, wobei R³ ggf. eine bis drei Gruppen R^{a} tragen kann. Insbesondere is R³ unsubstituiert.

Außerdem werden Verbindungen I besonders bevorzugt, in denen X für C₁-C₄-Alkyl, insbesondere Methyl steht.

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der *Ascomyceten, Deuteromyceten, Phycomyceten* und *Basidiomyceten,* aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankhei ten :
· *Alternaria*-Arten an Gemüse und Obst,
· *Bipolaris*- und Drechslera-Arten an Getreide, Reis und Rasen,
· *Blumeria graminis* (echter Mehltau) an Getreide,
· *Botrytis cinerea* (Grauschimmel) an Erdbeeren, Gemüse, Zierpflanzen und Reben,
· *Erysiphe cichoracearum und* Sphaerotheca fuliginea an Kürbisgewächsen,
· *Fusarium-* und *Verticillium-*Arten an verschiedenen Pflanzen,
· *Mycosphaerella*-Arten an Getreide, Bananen und Erdnüssen,
· *Phytophthora infestans* an Kartoffeln und Tomaten,
· *Plasmopara viticola* an Reben,
· *Podosphaera leucotricha* an Äpfeln,
· *Pseudocercosporella herpotrichoides* an Weizen und Gerste,
· *Pseudoperonospora-*Arten an Hopfen und Gurken,
· *Puccinia-*Arten an Getreide,
· *Pyricularia oryzae* an Reis,
· *Rhizoctonia*-Arten an Baumwolle, Reis und Rasen,
· *Septoria tritici* und Stagonospora nodorum an Weizen,
· *Uncinula necator* an Reben,
· *Ustilago-*Arten an Getreide und Zuckerrohr, sowie
· *Venturia*-Arten (Schorf) an Äpfeln und Birnen.

Die Verbindungen I eignen sich außerdem zur Bekämpfung von Schadpilzen wie *Paecilomyces variotii* im Materialschutz (z.B. Holz, Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2 kg Wirkstoff pro ha.

Bei der Saatgutbehandlung werden im allgemeinen Wirkstoffmengen von 0,001 bis 1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln. Als Lösungsmittel / Hilfsstoffe kommen dafür im wesentlichen in Betracht:
Wasser, aromatische Lösungsmittel (z.B. Solvesso Produkte, Xylol), Paraffine (z.B. Erdölfraktionen), Alkohole (z.B. Methanol, Butanol, Pentanol, Benzylalkohol), Ketone (z.B. Cyclohexanon, gamma-Butryolacton), Pyrrolidone (NMP, NOP), Acetate (Glykoldiacetat), Glykole, Dimethylfettsäureamide, Fettsäuren und Fettsäureester. Grundsätzlich können auch Lösungsmittelgemische verwendet werden,
Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate, Fettsäuren und sulfatierte Fettalkoholglykolether zum Einsatz, ferner Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Tristerylphenylpolyglykolether, Alkyl-arylpolyetheralkohole, Alkohol- und Fettalkoholethylenoxid-Kondensate, ethoxyliertes Rizinusöl, Polyoxyethylenalkylether, ethoxyliertes Polyoxypropylen, Laurylalkoholpoly-glykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
1. Produkte zur Verdünnung in Wasser
   A Wasserlösliche Konzentrate (SL)
      10 Gew.-Teile einer erfindungsgemäßen Verbindung werden in Wasser oder einem wasserlöslichen Lösungsmittel gelöst. Alternativ werden Netzmittel oder andere Hilfsmittel zugefügt. Bei der Verdünnung in Wasser löst sich der Wirkstoff.
   B Dispergierbare Konzentrate (DC)
      20 Gew.-Teile einer erfindungsgemäßen Verbindung werden in Cyclohexanon unter Zusatz eines Dispergiermittels z.B. Polyvinylpyrrolidon gelöst. Bei Verdünnung in Wasser ergibt sich eine Dispersion.
   C Emulgierbare Konzentrate (EC)
      15 Gew.-Teile einer erfindungsgemäßen Verbindung werden in Xylol unter Zusatz von Ca-Dodecylbenzolsulfonat und Ricinusölethoxylat (jeweils 5 %) gelöst. Bei der Verdünnung in Wasser ergibt sich eine Emulsion.
   D Emulsionen (EW, EO)
      40 Gew.-Teile einer erfindungsgemäßen Verbindung werden in Xylol unter Zusatz von Ca-Dodecylbenzolsulfonat und Ricinusölethoxylat (jeweils 5 %) gelöst. Diese Mischung wird mittels einer Emulgiermaschine (Ultraturax) in Wasser eingebracht und zu einer homogenen Emulsion gebracht. Bei der Verdünnung in Wasser ergibt sich eine Emulsion.
   E Suspensionen (SC, OD)
      20 Gew.-Teile einer erfindungsgemäßen Verbindung werden unter Zusatz von Dispergier- und Netzmitteln und Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs.
   F Wasserdispergierbare und wasserlösliche Granulate (WG, SG)
      50 Gew.-Teile einer erfindungsgemäßen Verbindung werden unter Zusatz von Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs.
   G Wasserdispergierbare und wasserlösliche Pulver (WP, SP)
      75 Gew.-Teile einer erfindungsgemäßen Verbindung werden unter Zusatz von Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs.
2. Produkte für die Direktapplikation
   H Stäube (DP)
      5 Gew.Teile einer erfindungsgemäßen Verbindung werden fein gemahlen und mit 95 % feinteiligem Kaolin innig vermischt. Man erhält dadurch ein Stäubemittel.
   I Granulate (GR, FG, GG, MG)
      0.5 Gew-Teile einer erfindungsgemäßen Verbindung werden fein gemahlen und mit 95.5 % Trägerstoffe verbunden. Gängige Verfahren sind dabei die Extrusion, die Sprühtrocknung oder die Wirbelschicht. Man erhält dadurch ein Granulat für die Direktapplikation.
   J ULV- Lösungen (UL)
      10 Gew.-Teile einer erfindungsgemäßen Verbindung werden in einem organischen Lösungsmittel z.B. Xylol gelöst. Dadurch erhält man ein Produkt für die Direktapplikation.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wässrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Netzmittel, Adjuvants, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
· Acylalanine wie Benalaxyl, Metalaxyl, Ofurace, Oxadixyl,
· Aminderivate wie Aldimorph, Dodine, Dodemorph, Fenpropimorph, Fenpropidin, Guazatine, Iminoctadine, Spiroxamin, Tridemorph
· Anilinopyrimidine wie Pyrimethanil, Mepanipyrim oder Cyrodinyl,
· Antibiotika wie Cycloheximid, Griseofulvin, Kasugamycin, Natamycin, Polyoxin oder Streptomycin,
· Azole wie Bitertanol, Bromoconazol, Cyproconazol, Difenoconazole, Dinitroconazol, Epoxiconazol, Fenbuconazol, Fluquiconazol, Flusilazol, Hexaconazol, Imazalil, Metconazol, Myclobutanil, Penconazol, Propiconazol, Prochloraz, Prothioconazol, Tebuconazol, Triadimefon, Triadimenol, Triflumizol, Triticonazol,
· Dicarboximide wie Iprodion, Myclozolin, Procymidon, Vinclozolin,
· Dithiocarbamate wie Ferbam, Nabam, Maneb, Mancozeb, Metam, Metiram, Propineb, Polycarbamat, Thiram, Ziram, Zineb,
. Heterocylische Verbindungen wie Anilazin, Benomyl, Boscalid, Carbendazim, Carboxin, Oxycarboxin, Cyazofamid, Dazomet, Dithianon, Famoxadon, Fenamidon, Fenarimol, Fuberidazol, Flutolanil, Furametpyr, Isoprothiolan, Mepronil, Nuarimol, Probenazol, Proquinazid, Pyrifenox, Pyroquilon, Quinoxyfen, Silthiofam, Thiabendazol, Thifluzamid, Thiophanat-methyl, Tiadinil, Tricyclazol, Triforine,
· Kupferfungizide wie Bordeaux Brühe, Kupferacetat, Kupferoxychlorid, basisches Kupfersulfat,
· Nitrophenylderivate, wie Binapacryl, Dinocap, Dinobuton, Nitrophthal-isopropyl
· Phenylpyrrole wie Fenpiclonil oder Fludioxonil,
· Schwefel
· Sonstige Fungizide wie Acibenzolar-S-methyl, Benthiavalicarb, Carpropamid, Chlorothalonil, Cyflufenamid, Cymoxanil, Dazomet, Diclomezin, Diclocymet, Diethofencarb, Edifenphos, Ethaboxam, Fenhexamid, Fentin-Acetat, Fenoxanil, Ferimzone, Fluazinam, Fosetyl, Fosetyl-Aluminium, Iprovalicarb, Hexachlorbenzol, Metrafenon, Pencycuron, Propamocarb, Phthalid, Toloclofos-methyl, Quintozene, Zoxamid
· Strobilurine wie Azoxystrobin, Dimoxystrobin, Fluoxastrobin, Kresoxim-methyl, Metominostrobin, Orysastrobin, Picoxystrobin, Pyraclostrobin oder Trifloxystrobin,
· Sulfensäurederivate wie Captafol, Captan, Dichlofluanid, Folpet, Tolylfluanid
· Zimtsäureamide und Analoge wie Dimethomorph, Flumetover oder Flumorph.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Angaben aufgeführt.

### Beispiel 1 Herstellung von 2-Cyclopentyl-3-oxobutylsäureethylester

Eine Mischung von 3-Oxobutylsäure ethylester (0,5 mol), Cyclopentylbromid (0,5 mol) und Natriumethoxylat (0,5 mol) in Ethanol (100 ml) wurde 15 Std. refluxiert. Nach Abkühlen der Reaktionsmischung auf etwa 20 bis 25°C wurden 71g der Titelverbindung durch Destillation (96-100°C, 0,25 mbar) isoliert.

### Beispiel 2 Herstellung von 5-Methyl-6-cyclopentyl-7-hydroxy-[1,2,4]-triazolo[1,5-a]-pyrimidin

Eine Mischung von 3-Amino-1,2,4-triazol (14 g), 2-Cyclopentyl-3-oxobutylsäureethylester (0,17 mol, Bsp. 1) und Tributylamin (50 ml) wurde für 6 Std. auf 180°C erhitzt. Nach Abkühlen der Reaktionsmischung auf etwa 70°C wurde wässr. NaOH-Lösung (21g in 200 ml H₂O) zugesetzt und die Lösung weitere 30 min gerührt. Nach Phasentrennung und Extraktion mit Diethylether wurde die wässrige Phase mit konz. HCl-Lösung angesäuert. Aus dem Niederschlag erhielt man 19g der Titelverbindung.

### Beispiel 3 Herstellung von 5-Methyl-6-cyclopentyl-7-chlor-[1,2,4]-triazolo[1,5-a]-pyrimidin

Eine Mischung von 5-Methyl-6-cyclopentyl-7-hydroxy-[1,2,4]-triazolo-[1,5-a]pyrimidin (17 g, Bsp. 2) und POCl₃ (50 ml) wurde 8 Std. refluxiert. Dabei destillierte ein Teil des POCl₃ ab. Der Rückstand wurde in ein Dichlormethan/Wasser-Gemisch gegeben. Die organische Phase wurde abgetrennt und getrocknet. Nach Abtrennen des Lösungsmittels erhielt man 11g der Titelverbindung vom Fp. 87°C.

### Beispiel 4 Herstellung von 5-Methyl-6-cyclopentyl-7-(4-methylpiperidin-1-yl)-[1,2,4]-triazolo[1,5-a] pyrimidin [I-1]

Eine Lösung von 4-Methylpiperidin (1,5 mmol) und Triethylamin (1,5 mmol) 10 ml Dichlormethan wurde unter Rühren zu einer Lösung von 5-Methyl-6-cyclopentyl-7-chlor-[1,2,4]-triazolo[1,5-a]-pyrimidin (1,5 mmol, Bsp. 3) in 20ml Dichlormethan gegeben. Die Reaktionsmischung wurde etwa 16 Std. bei 20 bis 25°C gerührt, dann mit 5%iger HCl-Lösung gewaschen. Die organische Phase wurde abgetrennt und getrocknet. Nach Abdestillieren des Lösungsmittels und Chromatographie an Kieselgel wurden daraus 0,26g der Titelverbindung vom Fp. 145°C erhalten.

**Tabelle I: Verbindungen der Formel 1**

| **Nr.** | **R**^{**1**} | **R**^{**2**} | **R**^{**3**} | **X** | **phys. Daten (Fp.[°C]; IR [cm**^{**-1**}**])** |
|---|---|---|---|---|---|
| I-1 | -(CH₂)₂CH(CH₃) (CH₂)₂- | | Cyclopentyl | CH₃ | 145 |
| I-2 | (R) CH (CH₃) -(CH(CH₃)₂ | H | Cyclopentyl | CH₃ | 96 |
| I-3 | (S) CH(CH3)-CF3 | H | Cyclopentyl | CH₃ | 108 |
| I-4 | H | H | Cyclopentyl | CH₃ | 271 |
| I-5 | CH₃ | H | Cyclopentyl | CH₃ | 175 |
| I-6 | (R) CH (CH₃)-C(CH₃)₃ | H | Cyclopentyl | CH₃ | 1960; 1605; 1590; 1240 |

### Beispiele für die Wirkung gegen Schadpilze

Die fungizide Wirkung der Verbindungen der Formel I ließ sich durch die folgenden Versuche zeigen:

Die Wirkstoffe wurden getrennt als Stammlösung formuliert mit 0,25 Gew.-% Wirkstoff in Aceton oder DMSO. Dieser Lösung wurde 1 Gew.-% Emulgator Uniperol® EL (Netzmittel mit Emulgier- und Dispergierwirkung auf der Basis ethoxylierter Alkylphenole) zugesetzt. Die Stammlösungen der Wirkstoffe wurden entsprechend der angegebenen Konzentration mit Wasser verdünnt.

### Beispiel 1: Wirksamkeit gegen die Dürrfleckenkrankheit der Tomate verursacht durch Alternaria solani

Blätter von Topfpflanzen der Sorte "Große Fleischtomate St. Pierre" wurden mit einer wässriger Suspension in der unten angegebenen Wirkstoffkonzentration bis zur Tropfnässe besprüht. Am folgenden Tag wurden die Blätter mit einer wässrigen Sporenaufschwemmung von *Alternaria solani* in 2 % Biomalzlösung mit einer Dichte von 0,17 x 10⁶ Sporen/ml infiziert. Anschließend wurden die Pflanzen in einer wasserdampfgesättigten Kammer bei Temperaturen zwischen 20 und 22°C aufgestellt. Nach 5 Tagen hatte sich die Krautfäule auf den unbehandelten, jedoch infizierten Kontrollpflanzen so stark entwickelt, dass der Befall visuell in % ermittelt werden konnte.

In diesem Test zeigten die mit 250 ppm des Wirkstoffs I-1 behandelten Pflanzen keinen Befall, während die unbehandelten Pflanzen zu 90% befallen war.

### Beispiel 2: Wirksamkeit gegen den Grauschimmel an Paprikablättern verursacht durch Botrytis cinerea bei protektiver Anwendung

Paprikasämlinge der Sorte "Neusiedler Ideal Elite" wurden, nachdem sich 4 - 5 Blätter gut entwickelt hatten, mit einer wässrigen Suspension in der unten angegebenen Wirkstoffkonzentration bis zur Tropfnässe besprüht. Am nächsten Tag wurden die behandelten Pflanzen mit einer Sporensuspension von *Botrytis cinerea,* die 1,7 x 10⁶ Sporen/ml in einer 2 %-igen wässrigen Biomalzlösung enthielt, inokuliert. Anschließend wurden die Versuchspflanzen in eine Klimakammer mit 22 bis 24°C und hoher Luftfeuchtigkeit gestellt. Nach 5 Tagen konnte das Ausmaß des Pilzbefalls auf den Blättern visuell in % ermittelt werden.

In diesem Test zeigten die mit 250 ppm der Wirkstoffe 1-1, bzw. I-3 behandelten Pflanzen maximal 20% Befall, während die unbehandelten Pflanzen zu 90% befallen waren.

### Beispiel 3 - Wirksamkeit gegen Mehltau an Gurkenblättern verursacht durch Sphaerotheca fuliginea bei protektiver Anwendung

Blätter von in Töpfen gewachsenen Gurkenkeimlingen der Sorte "Chinesische Schlange" wurden im Keimblattstadium mit wässriger Suspension in der unten angegebenen Wirkstoffkonzentration bis zur Tropfnässe besprüht. 20 Stunden nach dem Antrocknen des Spritzbelages wurden die Pflanzen mit einer wässrigen Sporensuspension des Gurkenmehltaus *(Sphaerotheca fuliginea)* inokuliert. Anschließend wurden die Pflanzen im Gewächshaus bei Temperaturen zwischen 20 und 24°C und 60 bis 80 % relativer Luftfeuchtigkeit für 7 Tage kultiviert. Dann wurde das Ausmaß der Mehltauentwicklung visuell in %-Befall der Keimblattfläche ermittelt.

In diesem Test zeigten die mit 250 ppm der Wirkstoffe I-1, bzw. I-3 behandelten Pflanzen nicht über 3% Befall, während die unbehandelten Pflanzen zu 90% befallen waren.

## Patentansprüche

1. 5-Alkyl-7-aminotriazolopyrimidine der Formel I, 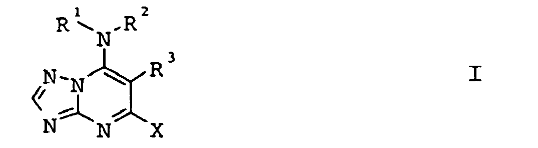 in der die Substituenten die folgenden Bedeutungen haben:
R¹,R² Wasserstoff, C₁-C₁₀-Alkyl, C₂-C₁₀-Alkenyl, C₂-C₁₀-Alkinyl, C₁-C₁₀-Haloalkyl, C₃-C₈-Cycloalkyl, Phenyl, Naphthyl; oder
5- oder 6-gliedriges gesättigtes, ungesättigtes oder aromatisches Heterocyclyl, enthaltend ein bis vier Stickstoffatome oder ein bis drei Stickstoffatome und ein Schwefel- oder Sauerstoffatom; oder
R¹ und R² können zusammen mit dem Stickstoffatom, das sie verbindet, einen 5- oder 6-gliedrigen Ring bilden, der ein bis vier Stickstoffatome oder ein bis drei Stickstoffatome und ein Schwefel- oder Sauerstoffatom enthält;
R¹ und R² können, wenn ungleich Wasserstoff, unabhängig voneinander teilweise oder vollständig halogeniert sein und/oder einen bis drei Reste aus der Gruppe R^{a} tragen
R^{a} Cyano, Nitro, Hydroxy, C₁-C₆-Alkyl, C₁-C₆-Haloalkyl, C₃-C₆-Cycloalkyl, C₁-C₆-Alkoxy, C₁-C₆-Haloalkoxy, C₁-C₆-Alkylthio, C₁-C₆-Alkylamino, Di-C₁-C₆-alkylamino, C₂-C₆-Alkenyl, C₂-C₆-Alkenyloxy, C₂-C₆-Alkinyl, C₃-C₆-Alkinyloxy und gegebenenfalls halogeniertes Oxy-C₁-C₄-alkylenoxy;
wobei diese aliphatischen oder alicyclischen Gruppen ihrerseits partiell oder vollständig halogeniert sein oder eine bis drei Gruppen R^{b} tragen können:
R^{b} Halogen, Cyano, Nitro, Hydroxy, Mercapto, Amino, Carboxyl, Aminocarbonyl, Aminothiocarbonyl, Alkyl, Haloalkyl, Alkenyl, Alkenyloxy, Alkinyloxy, Alkoxy, Halogenalkoxy, Alkylthio, Alkylamino, Dialkylamino, Formyl, Alkylcarbonyl, Alkylsulfonyl, Alkylsulfoxyl, Alkoxycarbonyl, Alkylcarbonyloxy, Alkylaminocarbonyl, Dialkylaminocarbonyl, Alkylaminothiocarbonyl, Dialkylaminothiocarbonyl, wobei die Alkylgruppen in diesen Resten 1 bis 6 Kohlenstoffatome enthalten und die genannten Alkenyl-oder Alkinylgruppen in diesen Resten 2 bis 8 Kohlenstoffatome enthalten;
und/oder einen bis drei der folgenden Reste:
Cycloalkyl, Cycloalkoxy, Heterocyclyl, Heterocyclyloxy, wobei die cyclischen Systeme 3 bis 10 Ringglieder enthalten; Aryl, Aryloxy, Arylthio, Aryl-C₁-C₆-alkoxy, Aryl-C₁-C₆-alkyl, Hetaryl, Hetaryloxy, Hetarylthio, wobei die Arylreste vorzugsweise 6 bis 10 Ringglieder, die Hetarylreste 5 oder 6 Ringglieder enthalten, wobei die cyclischen Systeme partiell oder vollständig halogeniert oder durch Alkyl-oder Haloalkylgruppen substituiert sein können;
R³ C₃-C₁₄-Cycloalkyl oder C₆-C₁₄-Bicycloalkyl, wobei R³ unsubstituiert oder teilweise oder vollständig halogeniert sein kann und/oder ein bis drei Reste aus der Gruppe R^{a} tragen kann; und
X C₁-C₆-Alkyl oder C₁-C₂-Halogenalkyl;
sowie deren Salze.

2. 5-Alkyl-7-aminotriazolopyrimidine der Formel I gemäß Anspruch 1, in der die Substituenten die folgenden Bedeutungen haben:
R¹,R² Wasserstoff, C₁-C₁₀-Alkyl, C₂-C₁₀-Alkenyl, C₂-C₁₀-Alkinyl, C₁-C₁₀-Haloalkyl, C₃-C₈-Cycloalkyl, Phenyl, Naphthyl; oder
5- oder 6-gliedriges gesättigtes, ungesättigtes oder aromatisches Heterocyclyl, enthaltend ein bis vier Stickstoffatome oder ein bis drei Stickstoffatome und ein Schwefel- oder Sauerstoffatom; oder
R¹ und R² können zusammen mit dem Stickstoffatom, das sie verbindet, einen 5- oder 6-gliedrigen Ring bilden, der ein bis vier Stickstoffatome oder ein bis drei Stickstoffatome und ein Schwefel- oder Sauerstoffatom enthält;
R¹ und R² können, wenn ungleich Wasserstoff, unabhängig voneinander teilweise oder vollständig halogeniert sein und/oder einen bis drei Reste aus der Gruppe R^{a} tragen
R^{a} Cyano, Nitro, Hydroxy, C₁-C₆-Alkyl, C₁-C₆-Haloalkyl, C₃-C₆-Cycloalkyl, C₁-C₆-Alkoxy, C₁-C₆-Haloalkoxy, C₁-C₆-Alkylthio, C₁-C₆-Alkylamino, Di-C₁-C₆-alkylamino, C₂-C₆-Alkenyl, C₂-C₆-Alkenyloxy, C₂-C₆-Alkinyl, C₃-C₆-Alkinyloxy und gegebenenfalls halogeniertes Oxy-C₁-C₄-alkylenoxy;
R³ C₃-C₁₄-Cycloalkyl oder C₆-C₁₄-Bicycloalkyl, wobei R³ unsubstituiert oder teilweise oder vollständig halogeniert sein kann und/oder ein bis drei Reste aus der Gruppe R^{a} tragen kann; und
X C₁-C₆-Alkyl;
sowie deren Salze.

3. Verbindungen der Formel I gemäß Anspruch 1 oder 2, in der X für Methyl steht.

4. Verfahren zur Herstellung von Verbindungen der Formel I nach Anspruch 1, **dadurch gekennzeichnet, daß** man Dicarbonylverbindungen der Formel II 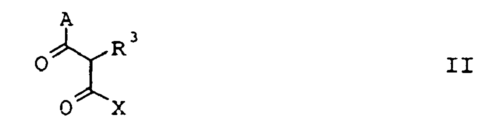 wobei A für C₁-C₁₀-Alkoxy und R³ und X wie für Formel I definiert ist, mit 3-Amino-1,2,4-triazol zu 7-Hydroxytriazolopyrimidinen der Formel III 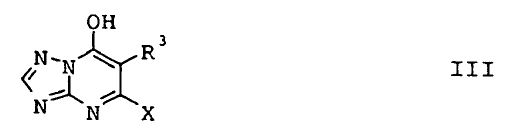 cyclisiert und III mit einem Halogenierungsmittel zu 7-Halogentriazolopyrimidinen der Formel IV 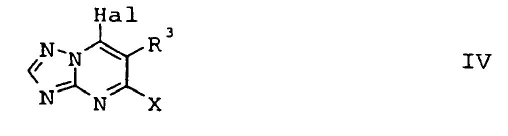 halogeniert, wobei Hal für Halogen steht, und anschließend mit einem Amin der Formel V 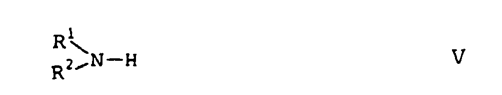 worin R¹ und R² wie in Formel I definiert sind, zu 5-Alkyl-7-aminotriazolopyrimidinen der Formel I umsetzt.

5. Verbindungen der Formeln III und IV wie in Anspruch 4 definiert.

6. Zur Bekämpfung von phytopathogenen Schadpilzen geeignetes Mittel, enthaltend einen festen oder flüssigen Trägerstoff und eine Verbindung der Formel I gemäß Anspruch 1.

7. Saatgut, enthaltend eine Verbindung der Formel I gemäß Anspruch 1 in einer Menge von 0,001 bis 1 g/kg.

8. Verwendung der Verbindungen der Formel I gemäß Anspruch 1 zur Herstellung eines zur Bekämpfung von Schadpilzen geeigneten Mittels.

9. Verfahren zur Bekämpfung von phytopathogenen Schadpilzen, **dadurch gekennzeichnet, daß** man die Pilze oder die vor Pilzbefall zu schützenden Materialien, Pflanzen, den Boden oder Saatgüter mit einer wirksamen Menge der Verbindungen der Formel I gemäß Anspruch 1 behandelt.

## Claims

1. A 5-alkyl-7-aminotriazolopyrimidine of the formula I, 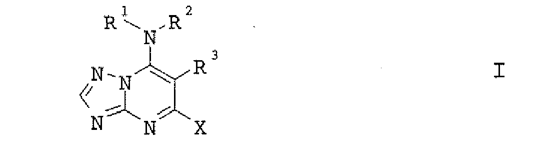 where:
R¹,R² are hydrogen, C₁-C₁₀-alkyl, C₂-C₁₀-alkenyl, C₂-C₁₀₋alkynyl, C₁-C₁₀-haloalkyl, C₃-C₈-cycloalkyl, phenyl, naphthyl; or
5- or 6-membered saturated, unsaturated or aromatic heterocyclyl which contains one to four nitrogen atoms or one to three nitrogen atoms and one sulfur or oxygen atom; or
R¹ and R² together with the bridging nitrogen atom can form a 5- or 6-membered ring which contains one to four nitrogen atoms or one to three nitrogen atoms and one sulfur or oxygen atom;
if R¹ and R² are not hydrogen they can, independently of one another, be partially or fully halogenated and/or may carry one to three radicals from the group R^{a}
R^{a} is cyano, nitro, hydroxyl, C₁-C₆-alkyl, C₁-C₆₋haloalkyl, C₃-C₆-cycloalkyl, C₁-C₆-alkoxy, C₁-C₆₋haloalkoxy, C₁-C₆-alkylthio, C₁-C₆-alkylamino, di-C₁-C₆-alkylamino, C₂-C₆-alkenyl, C₂-C₆₋alkenyloxy, C₂-C₆-alkynyl, C₃-C₆-alkynyloxy and unhalogenated or halogenated oxy-C₁-C₄-alkyleneoxy;
where these aliphatic, or alicyclic, groups for their part may be partially or fully halogenated or may carry one to three groups R^{b}:
R^{b} is halogen, cyano, nitro, hydroxyl, mercapto, amino, carboxyl, aminocarbonyl, aminothiocarbonyl, alkyl, haloalkyl, alkenyl, alkenyloxy, alkynyloxy, alkoxy, haloalkoxy, alkylthio, alkylamino, dialkylamino, formyl, alkylcarbonyl, alkyl-sulfonyl, alkylsulfoxyl, alkoxycarbonyl, alkylcarbonyloxy, alkylaminocarbonyl, dialkyl-aminocarbonyl, alkylaminothiocarbonyl, dialkyl-aminothiocarbonyl, where the alkyl groups in these radicals contain 1 to 6 carbon atoms and the alkenyl or alkynyl groups mentioned in these radicals contain 2 to 8 carbon atoms;
and/or one to three of the following radicals:
cycloalkyl, cycloalkoxy, heterocyclyl, heterocyclyloxy, where the cyclic systems contain 3 to 10 ring members; aryl, aryloxy, arylthio, aryl-C₁-C₆-alkoxy, aryl-C₁-C₆-alkyl, hetaryl, hetaryloxy, hetarylthio, where the aryl radicals preferably contain 6 to 10 ring members, the hetaryl radicals contain 5 or 6 ring members, where the cyclic systems may be partially or fully halogenated or may be substituted by alkyl or haloalkyl groups;
R³ is C₃-C₁₄-cycloalkyl or C₆-C₁₄-bicycloalkyl, where R³ may be unsubstituted or partially or fully halogenated and/or may carry one to three radicals from the group R^{a}; and
X is C₁-C₆-alkyl or C₁-C₂-haloalkyl;
and its salts.

2. The 5-alkyl-7-aminotriazolopyrimidine of the formula I according to claim 1 where:
R¹,R² are hydrogen, C₁-C₁₀-alkyl, C₂-C₁₀-alkenyl, C₂-C₁₀₋alkynyl, C₁-C₁₀-haloalkyl, C₃-C₈-cycloalkyl, phenyl, naphthyl; or
5- or 6-membered saturated, unsaturated or aromatic heterocyclyl which contains one to four nitrogen atoms or one to three nitrogen atoms and one sulfur or oxygen atom; or
R¹ and R² together with the bridging nitrogen atom can form a 5- or 6-membered ring which contains one to four nitrogen atoms or one to three nitrogen atoms and one sulfur or oxygen atom;
if R¹ and R² are not hydrogen they can, independently of one another, be partially or fully halogenated and/or may carry one to three radicals from the group R^{a}
R^{a} is cyano, nitro, hydroxyl, C₁-C₆-alkyl, C₁-C₆₋haloalkyl, C₃-C₆-cycloalkyl, C₁-C₆-alkoxy, C₁-C₆₋haloalkoxy, C₁-C₆-alkylthio, C₁-C₆-alkylamino, di-C₁-C₆-alkylamino, C₂-C₆-alkenyl, C₂-C₆₋alkenyloxy, C₂-C₆-alkynyl, C₃-C₆-alkynyloxy and unhalogenated or halogenated oxy-C₁-C₄-alkyleneoxy;
R³ is C₃-C₁₄-cycloalkyl or C₆-C₁₄-bicycloalkyl, where R³ may be unsubstituted or partially or fully halogenated and/or may carry one to three radicals from the group R^{a}; and
X is C₁-C₆-alkyl;
and its salts.

3. The compound of the formula I according to claim 1 or 2 in which X is methyl.

4. A process for preparing compounds of the formula I according to claim 1, which comprises cyclizing dicarbonyl compounds of the formula II 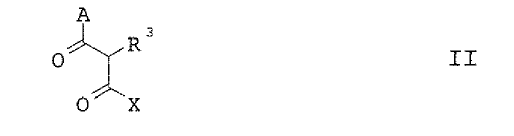 where A is C₁-C₁₀-alkoxy and R³ and X are as defined for formula I with 3-amino-1,2,4-triazole to give 7-hydroxytriazolopyrimidines of the formula III 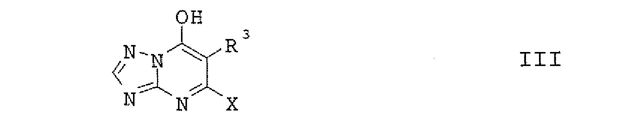 and halogenating III with a halogenating agent to give 7-halogentriazolopyrimidines of the formula IV 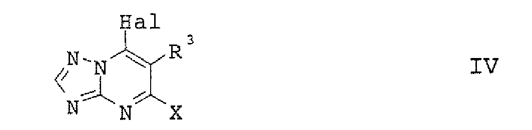 where Hal is halogen, followed by reaction with an amine of the formula V 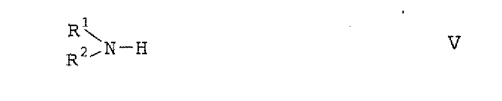 where R¹ and R² are as defined in formula I, to give 5-alkyl-7-aminotriazolopyrimidines of the formula I.

5. A compound of formula III or IV as defined in claim 4.

6. A composition suitable for controlling phytopathogenic harmful fungi, which composition comprises a solid or liquid carrier and a compound of the formula I according to claim 1.

7. Seeds, comprising a compound of the formula I according to claim 1 in an amount of from 0.001 to 1 g/kg.

8. The use of the compounds of the formula I according to claim 1 for preparing a composition suitable for controlling harmful fungi.

9. A method for controlling phytopathogenic harmful fungi, which comprises treating the fungi or the materials, plants, the soil or the seeds to be protected against fungal attack with an effective amount of a compound of the formula I according to claim 1.

## Revendications

1. 5-Alkyl-7-aminotriazolopyrimidines de la formule I : 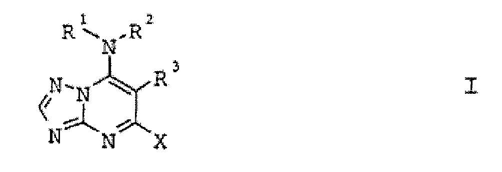 dans laquelle les substituants ont les significations suivantes:
R¹, R² représentent de l'hydrogène ou un groupe alkyle en C₁-C₁₀, alcényle en C₂-C₁₀, alcynyle en C₂-C₁₀, halogénoalkyle en C₁-C₁₀, cycloalkyle en C₃-C₈, phényle, naphtyle, ou
un groupe hétérocyclyle à 5 ou 6 termes, saturé, insaturé ou aromatique, contenant un à quatre atomes d'azote ou un à trois atomes d'azote et un atome de soufre ou d'oxygène, ou
R¹ et R² peuvent former conjointement avec l'atome d'azote qui les relie un noyau pentagonal ou hexagonal qui contient un à quatre atomes d'azote ou un à trois atomes d'azote et un atome de soufre ou d'oxygène,
R¹ et R² pouvant, lorsqu'ils sont différents de l'hydrogène, être partiellement ou totalement halogénés, indépendamment l'un de l'autre, et/ou porter un à trois radicaux du groupe R^{a},
R^{a} représentant un groupe cyano, nitro, hydroxy, alkyle en C₁-C₆, halogénoalkyle en C₁-C₆, cycloalkyle en C₃-C₆, alcoxy en C₁-C₆, halogénoalcoxy en C₁-C₆, alkylthio en C₁-C₆, alkylamino en C₁-C₆, dialkylamino en C₁-C₆, alcényle en C₂-C₆, alcényloxy en C₂-C₆, alcynyle en C₂-C₆, alcynyloxy en C₂-C₆ et oxyalkylénoxy en C₁-C₄ éventuellement halogéné,
ces groupes aliphatiques ou alicycliques pouvant à leur tour être partiellement ou totalement halogénés ou porter un à trois groupes R^{b},
R^{b} représentant de l'halogène ou un groupe cyano, nitro, hydroxy, mercapto, amino, carboxyle, aminocarbonyle, aminothiocarbonyle, alkyle, halogénoalkyle, alcényle, alcényloxy, alcynyloxy, alcoxy, halogénoalcoxy, alkylthio, alkylamino, dialkylamino, formyle, alkylcarbonyle, alkylsulfonyle, alkylsulfoxyle, alcoxycarbonyle, alkylcarbonyloxy, alkylaminocarbonyle, dialkylaminocarbonyle, alkylaminothiocarbonyle, dialkylaminothiocarbonyle, les groupes alkyle dans ces radicaux contenant 1 à 6 atomes de carbone et les groupes alcényle ou alcynyle cités contenant dans ces radicaux 2 à 8 atomes de carbone,
et/ou un à trois des radicaux suivants :
cycloalkyle, cycloalcoxy, hétérocyclyle, hétérocyclyloxy, les systèmes cycliques contenant 3 à 10 termes ; aryle, aryloxy, arylthio, arylalcoxy en C₁-C₆, arylalkyle en C₁-C₆, hétaryle, hétaryloxy, hétarylthio, les radicaux aryle contenant de préférence 6 à 10 termes, les radicaux hétaryle 5 ou 6 termes, les systèmes cycliques pouvant être partiellement ou totalement halogénés ou être substitués par des groupes alkyle ou halogénoalkyle,
R³ représente un groupe cycloalkyle en C₃-C₁₄ ou bicycloalkyle en C₆-C₁₄, R³ pouvant être non substitué ou partiellement ou totalement halogéné et/ou porter un à trois radicaux du groupe R^{a}, et
X représente un groupe alkyle en C₁-C₆ ou halogénoalkyle en C₁-C₂,
ainsi que leurs sels.

2. 5-Alkyl-7-aminotriazolopyrimidines de la formule I suivant la revendication 1, dans laquelle les substituants ont les significations suivantes :
R¹, R² représentent de l'hydrogène ou un groupe alkyle en C₁-C₁₀, alcényle en C₂-C₁₀, alcynyle en C₂-C₁₀, halogénoalkyle en C₁-C₁₀, cycloalkyle en C₃-C₈, phényle, naphtyle, ou
un groupe hétérocyclyle à 5 ou 6 termes, saturé, insaturé ou aromatique, contenant un à quatre atomes d'azote ou un à trois atomes d'azote et un atome de soufre ou d'oxygène, ou
R¹ et R² peuvent former conjointement avec l'atome d'azote qui les relie un noyau pentagonal ou hexagonal qui contient un à quatre atomes d'azote ou un à trois atomes d'azote et un atome de soufre ou d'oxygène,
R¹ et R² pouvant, lorsqu'ils sont différents de l'hydrogène, être partiellement ou totalement halogénés, indépendamment l'un de l'autre, et/ou porter un à trois radicaux du groupe R^{a},
R^{a} représentant un groupe cyano, nitro, hydroxy, alkyle en C₁-C₆, halogénoalkyle en C₁-C₆, cycloalkyle en C₃-C₆, alcoxy en C₁-C₆, halogénoalcoxy en C₁-C₆, alkylthio en C₁-C₆, alkylamino en C₁-C₆, dialkylamino en C₁-C₆, alcényle en C₂-C₆, alcényloxy en C₂-C₆, alcynyle en C₂-C₆, alcynyloxy en C₃-C₆ et oxyalkylénoxy en C₁-C₄ éventuellement halogéné,
R³ représente un groupe cycloalkyle en C₃-C₁₄ ou bicycloalkyle en C₆-C₁₄, R³ pouvant être non substitué ou partiellement ou totalement halogéné et/ou pouvant porter un à trois radicaux du groupe R^{a}, et
X représente un groupe alkyle en C₁-C₆,
ainsi que leurs sels.

3. Composés de la formule I suivant la revendication 1 ou 2, dans laquelle X représente du méthyle.

4. Procédé de préparation de composés de la formule I suivant la revendication 1, **caractérisé en ce qu'**on cyclise des composés dicarbonyle de la formule II : 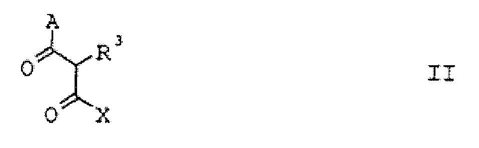 dans laquelle A représente un groupe alcoxy en C₁-C₁₀ et R³ et X sont définis comme dans la formule I, avec du 3-amino-1,2,4-triazole en 7-hydroxytriazolopyrimidines de la formule III : 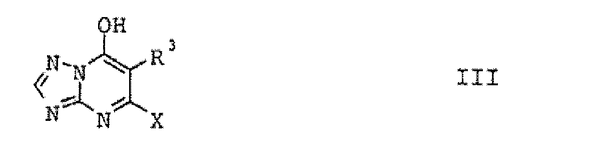 et on halogène le composé III avec un agent d'halogénation pour former des 7-halogénotriazolopyrimidines de la formule IV : 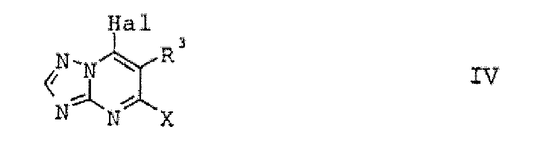 dans laquelle Hal représente de l'halogène, et ensuite on fait réagir avec une amine de la formule V : 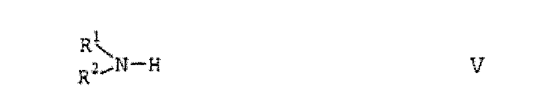 dans laquelle R¹ et R² sont définis comme dans la formule I, pour former des 5-alkyl-7-aminotriazolopyrimidines de la formule 1.

5. Composés des formules III et IV, tels que définis dans la revendication 4.

6. Produits appropriés pour lutter contre des champignons nuisibles phytopathogènes, contenant un support solide ou liquide et un composé de la formule I suivant la revendication 1.

7. Semences, contenant un composé de la formule I suivant la revendication 1 en une quantité de 0,001 à 1 g/kg.

8. Utilisation des composés de la formule I suivant la revendication 1, pour la préparation d'un produit approprié pour lutter contre les champignons nuisibles.

9. Procédé de lutter contre des champignons nuisibles phytopathogènes, **caractérisé en ce qu'**on traite les champignons ou les matériels, plantes, sols ou semences à protéger d'une infection par des champignons avec une quantité efficace des composés de la formule I suivant la revendication 1.
